# EUROPEAN PATENT SPECIFICATION

(11) **EP 4 415 795 B1**
(45) Date of publication and mention of the grant of the patent: **01.07.2026**
(21) Application number: 22812891.4
(22) Date of filing: 21.10.2022
(51) Int. Cl.: A61M 25/06, A61M 25/09, A61M 25/00

(54) **GUIDEWIRE LOCKING SYSTEM FOR CATHETER PLACEMENT**
FÜHRUNGSDRAHTVERRIEGELUNGSSYSTEM FÜR KATHETERPLATZIERUNG
SYSTÈME DE VERROUILLAGE DE FIL-GUIDE POUR PLACEMENT DE CATHÉTER

(30) Priority: 21.10.2021 US 202163270491 P
(43) Date of publication of application: 21.08.2024
(73) Proprietor: Bard Access Systems, Inc., Salt Lake City, UT 84116 (US)
(72) Inventor: LINDEKUGEL, Eric W., Salt Lake City, UT 84106 (US); BLANCHARD, Daniel B., Bountiful, UT 84010 (US)
(74) Representative: Hoffmann Eitle
(86) International application number: PCT/US2022/047444
(87) International publication number: WO 2023/069726

(56) References cited:
- EP-A1- 0 641 571
- EP-A1- 3 205 368
- WO-A1-93/06878
- US-A- 4 935 008
- US-A1- 2009 105 653
- US-A1- 2021 085 927

## Description

### PRIORITY

This application claims the benefit of priority to U.S. Provisional Application No. 63/270,491, filed October 21, 2021.

### BACKGROUND

US 2021/0085927 A1 discloses a catheter assembly including a peripheral intravenous ("PIV") portion that provides expedited vascular access. The catheter assembly includes an elongated catheter body defining a first lumen and includes a CVC portion, a transition portion, and a PIV portion. The PIV portion defines a relatively smaller diameter, a tapered tip, and a lumen configured to receive a needle. The needle extends through a needle access aperture, disposed through a side wall of the catheter body, through the PIV lumen, to a point beyond a distal end of the catheter body. A clinician accesses a vasculature using the needle and the PIV portion. The needle is then removed and a guidewire advanced through the lumen of the catheter body and the PIV portion into the vasculature. The CVC portion is then advanced over the guidewire into the vasculature, self-dilating the insertion site as the transition portion enters the vasculature.

### SUMMARY

According to the invention, there is provided a catheter placement system in accordance with claim 1. The dependent claims define preferred embodiments.

Briefly summarized, embodiments disclosed herein are directed to a guidewire locking system for a Rapidly Inserted Central Catheter (RICC) placement system, and associated methods thereof. When placing a catheter, e.g. a RICC catheter, it can be advantageous to obtain and stabilize venous access as soon as possible after venipuncture. To achieve this, a distal tip of a guidewire can reside within a needle lumen during venipuncture. Once venous access has been confirmed, the guidewire can be advanced into the vasculature to maintain patency of the access site. The needle can then be removed, preferably in such a way so as to leave the guidewire in place.

Some RICC placement systems utilize a slotted needle including a sheath disposed thereover. A distal tip of the guidewire can remain in position within the vasculature and a portion of the guidewire can pass through the needle slot as the needle is withdrawn proximally. The sheath can cover the slot and maintain the integrity of the needle lumen, e.g. to prevent fluid leakage through the slot. As the guidewire passes through the needle slot the guidewire can tear the sheath. However, as the guidewire passes through the needle slot, forces acting on the guidewire can dislodge the position of the distal tip within the vasculature. Embodiments disclosed herein are directed to a guidewire locking system configured to couple with a RICC catheter placement system and stabilize the guidewire in position as the needle is withdrawn proximally from the access site.

Disclosed herein is a catheter placement system including, a needle supported by a needle hub and defining a needle lumen, a guidewire having a distal tip disposed within the needle lumen, a catheter disposed on a proximal portion of the guidewire, and a guidewire locking system including, a housing defining a needle channel and a guidewire channel communicating with the needle channel and extending at an angle thereto, a portion of the needle extending through the needle channel and a portion of the guidewire extending through the guidewire channel, and a locking mechanism having a lock and a seal and transitionable between an unlocked position and a locked position, the locking mechanism in the locked position configured to inhibit axial movement of the guidewire through the guidewire channel.

In some embodiments, the seal is formed of a compliant material and defines a seal guidewire lumen configured to receive a portion of the guidewire therethrough, the lock in the locked position configured to compress the seal radially inwards to impinge on the guidewire and inhibit axial movement thereof. In some embodiments, a diameter of the seal guidewire lumen is equal to or less than an outer diameter of the guidewire and engages the guidewire in an interference fit in one or both of the locked position and the unlocked position. In some embodiments, the lock includes a tapered distal end configured to receive a portion to the seal therein and compress the seal radially inwards as the locking mechanism is transitioned to the locked position.

In some embodiments, the lock includes a collar threadably engaged with the housing and configured to urge the lock axially through the guidewire channel as the lock is rotated about the axis of the guidewire. In some embodiments, the collar includes a lever extending therefrom, perpendicular to an axis of the guidewire channel. In some embodiments, the catheter placement system further includes a peel pin extending through the seal and disposed at a junction between the guidewire channel and the needle channel and configured to support the guidewire as the needle is withdrawn proximally from the needle channel. In some embodiments, the peel pin is formed integrally with the housing. In some embodiments, the peel pin is formed as a separate structure and coupled with the housing using one of an interference fit, press-fit, snap-fit engagement, adhesive, bonding, or welding.

In some embodiments, a proximal end of the housing is releasably engaged with the needle hub in one of an interference fit, press fit, or snap fit engagement. In some embodiments, the catheter placement system further includes a handle extending from the housing and configured to facilitate grasping and manipulating the housing relative to the needle hub. In some embodiments, the housing includes a housing slot extending longitudinally and communicating with one or both of the needle channel and the guidewire channel, the housing slot configured to allow the guidewire to pass therethrough when the locking mechanism is in the unlocked position.

In some embodiments, the seal includes a seal slot extending longitudinally and communicating with one or both of the seal guidewire lumen and a seal needle lumen, the seal slot configured to allow the guidewire to pass therethrough when the locking mechanism is in the unlocked position. In some embodiments, the lock includes a lock slot extending longitudinally and communicating with a lock lumen, the lock slot configured to align with the housing slot in the unlocked position and allow the guidewire to pass therethrough to disengage the guidewire locking system. In some embodiments, the needle includes a guidewire aperture disposed in a side wall of the needle and configured to receive a portion of the guidewire therethrough, the needle also including a needle slot extending longitudinally between the guidewire aperture and a distal tip of the needle and communicating with a needle lumen, the needle slot configured to receive a portion of the guidewire therethrough as the needle is withdrawn proximally.

In some embodiments, the catheter placement system further includes a sheath encircling a portion of the needle and including a tear line extending longitudinally between a proximal end and a distal end of the sheath. In some embodiments, the lock includes a compression piece rotatably coupled to a distal end of a rotation piece, the rotation piece configured to rotate about an axis of the guidewire channel and urge the compression piece axially. In some embodiments, the compression piece includes a rail extending from an outer surface thereof and configured to engage a recess disposed in an inner wall of the guidewire channel to mitigate rotational movement of the compression piece while allowing axial movement thereof.

In some embodiments, the locking mechanism includes an actuator slidably engaged with the housing along an axis extending perpendicular to an axis of the guidewire channel, the actuator transitionable between the locked and unlocked position. In some embodiments, the locking mechanism includes a push-button mechanism having a first force applied in a first direction to transition the actuator from the unlocked position to the locked position and a second force also applied in the first direction to transition the actuator from the locked position to the unlocked position.

Also disclosed, but not encompassed by the claims, is a method of placing a catheter including, accessing a vasculature using a needle supported by a needle hub, advancing a guidewire through a needle lumen of the needle and into the vasculature, locking the guidewire using a guidewire locking system having a housing defining a needle channel and a guidewire channel communicating with the needle channel and extending at an angle thereto, the housing releasably coupled to a distal end of the needle hub, disengaging the housing from the needle hub, withdrawing the needle proximally from the needle channel of the housing, unlocking the guidewire stabilizing system to allow axial movement of the guidewire relative to the housing, disengaging the guidewire from the housing by sliding the guidewire through a housing slot disposed in a side wall of the housing, and advancing the catheter over the guidewire and into the vasculature.

In some embodiments, withdrawing the needle proximally further includes sliding a portion of the guidewire through a needle slot, from a guidewire aperture disposed in a side wall of the needle adjacent the needle hub, to a distal tip of the needle. In some embodiments, withdrawing the needle proximally further includes tearing a sheath along a tear line the sheath encircling a portion of the needle. In some embodiments, a distal tip of the guidewire is disposed within the needle lumen prior to accessing the vasculature.

In some embodiments, locking the guidewire further comprises, rotating a collar coupled to a lock and threadably engaged with the housing, advancing the lock axially through the guidewire channel, and compressing a seal radially inwards to grip the guidewire extending therethrough and prevent axial movement of the guidewire relative to the housing. In some embodiments, advancing the lock axially further includes compressing a portion of the seal radially inwards, the portion of the seal when the lock is advanced axially extending into a recess disposed at a distal end of the lock.

In some embodiments, rotating the collar includes rotating a rotation piece coupled thereto and urging a compression piece axially, the compression piece rotatably coupled to the rotation piece. In some embodiments, the method further includes engaging a rail, extending from an outer surface of the compression piece, with a recess disposed in an inner wall of the guidewire channel of the housing, to prevent the compression piece from rotating about an axis of the guidewire, while allowing the compression piece to slide axially relative to the axis of the guidewire. In some embodiments, the collar includes a lever extending therefrom perpendicular to an axis of the guidewire.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a perspective view of an exemplary RICC placement system, in accordance with embodiments disclosed herein.
FIG. 1B shows a plan view of a needle, sheath and guidewire assembly of the RICC placement system of FIG. 1A, in accordance with embodiments disclosed herein.
FIG. 1C shows a plan view of a needle of the RICC placement system of FIG. 1A, in accordance with embodiments disclosed herein.
FIG. 2A shows a cross-section view of a guidewire locking system, in accordance with embodiments disclosed herein.
FIG. 2B shows a perspective view of a guidewire locking system in a locked position, in accordance with embodiments disclosed herein.
FIG. 2C shows a perspective view of a guidewire locking system in an unlocked position, in accordance with embodiments disclosed herein.
FIG. 3A shows a perspective view of a guidewire locking system in a locked position, in accordance with embodiments disclosed herein.
FIG. 3B shows a perspective view of a guidewire locking system in an unlocked position, in accordance with embodiments disclosed herein.
FIG. 4A shows a cross-section view of a guidewire locking system, in accordance with embodiments disclosed herein.
FIG. 4B shows a lateral cross-section view of the guidewire locking system of FIG. 4A, in accordance with embodiments disclosed herein.
FIG. 5 shows a cross-section view of a guidewire locking system, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

In the following description, the terms "or" and "and/or" as used herein are to be interpreted as inclusive or meaning any one or any combination. As an example, "A, B or C" or "A, B and/or C" mean "any of the following, A, B, C, A and B, A and C, B and C, A, B and C." An exception to this definition will occur only when a combination of elements, components, functions, steps or acts are in some way inherently mutually exclusive.

With respect to "proximal," a "proximal portion" or a "proximal end portion" of, for example, a needle disclosed herein includes a portion of the needle intended to be near a clinician when the needle is used on a patient. Likewise, a "proximal length" of, for example, the needle includes a length of the needle intended to be near the clinician when the needle is used on the patient. A "proximal end" of, for example, the needle includes an end of the needle intended to be near the clinician when the needle is used on the patient. The proximal portion, the proximal end portion, or the proximal length of the needle can include the proximal end of the needle, however, the proximal portion, the proximal end portion, or the proximal length of the needle need not include the proximal end of the needle. That is, unless context suggests otherwise, the proximal portion, the proximal end portion, or the proximal length of the needle is not a terminal portion or terminal length of the needle.

With respect to "distal," a "distal portion" or a "distal end portion" of, for example, a needle disclosed herein includes a portion of the needle intended to be near or in a patient when the needle is used on the patient. Likewise, a "distal length" of, for example, the needle includes a length of the needle intended to be near or in the patient when the needle is used on the patient. A "distal end" of, for example, the needle includes an end of the needle intended to be near or in the patient when the needle is used on the patient. The distal portion, the distal end portion, or the distal length of the needle can include the distal end of the needle, however, the distal portion, the distal end portion, or the distal length of the needle need not include the distal end of the needle. That is, unless context suggests otherwise, the distal portion, the distal end portion, or the distal length of the needle is not a terminal portion or terminal length of the needle.

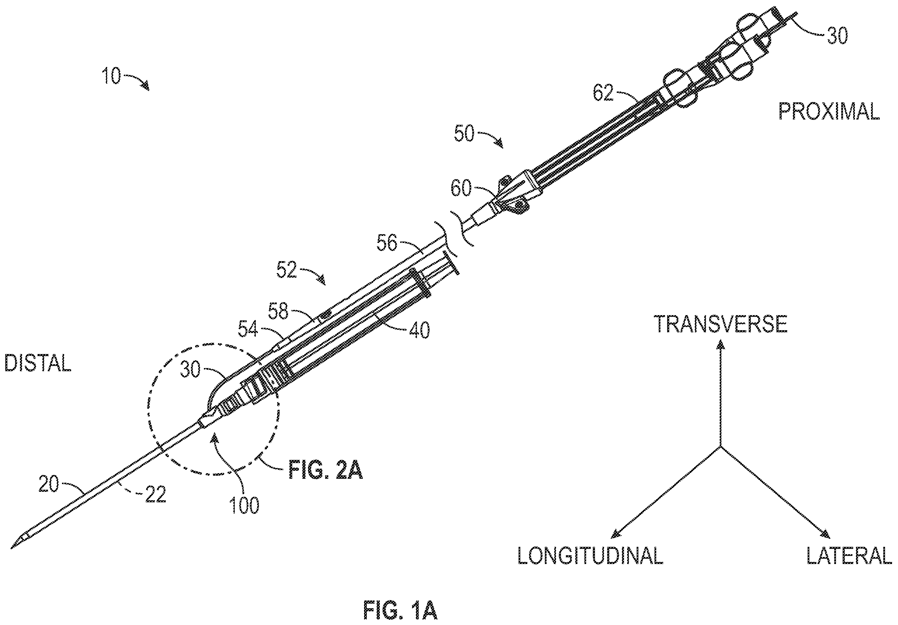

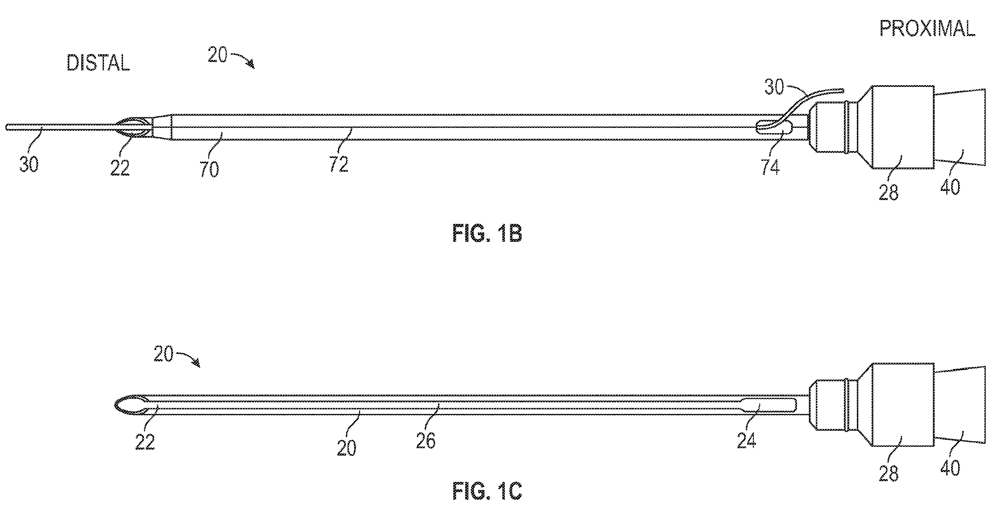

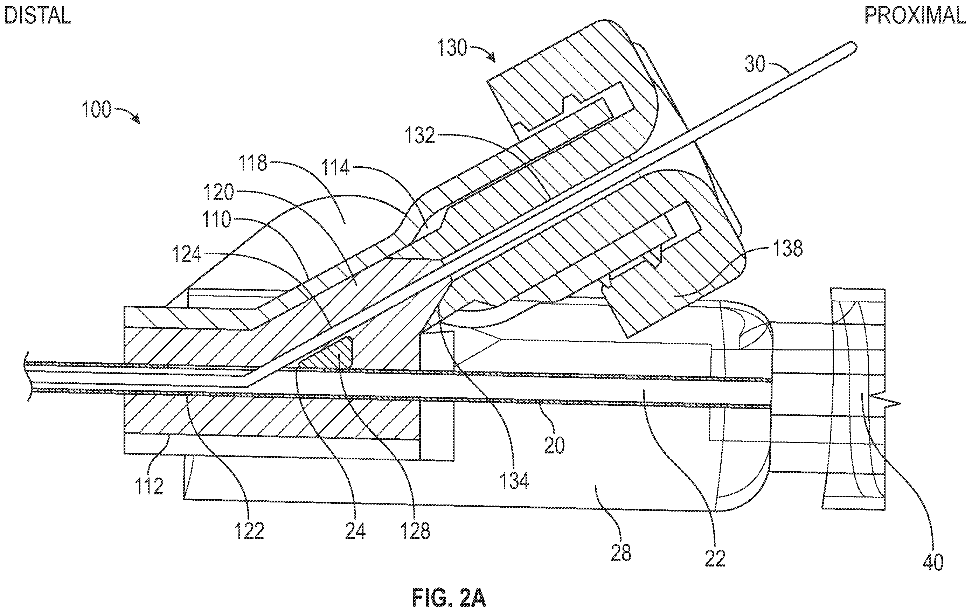

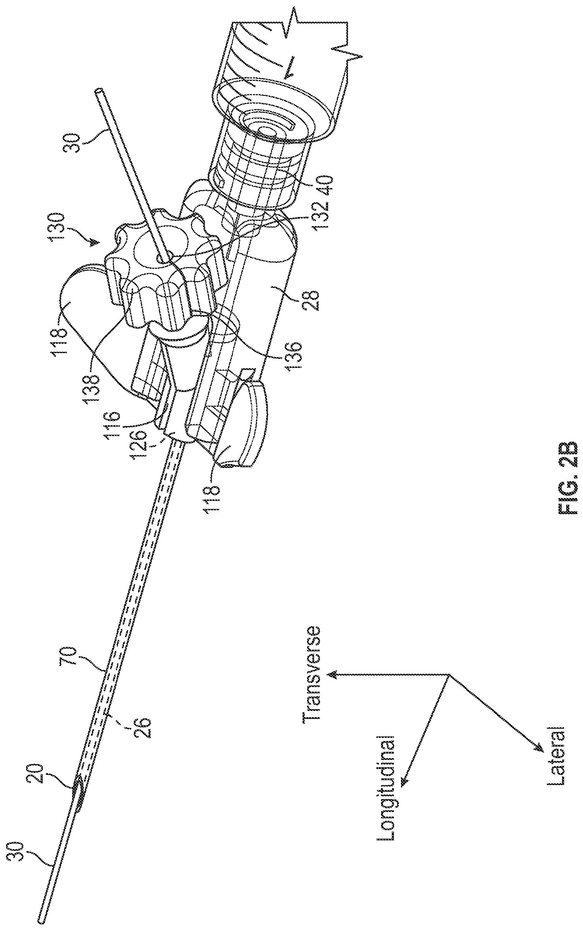

To assist in the description of embodiments described herein, as shown in FIGS. 1A, 2B, a longitudinal axis extends substantially parallel to an axis of the needle 20. A lateral axis extends normal to the longitudinal axis, and a transverse axis extends normal to both the longitudinal and lateral axes.

Unless defined otherwise, all technical and scientific terms used herein have the same meaning as commonly understood by those of ordinary skill in the art.

FIGS. 1A-1C show details of an exemplary Rapidly Insertable Central Catheter (RICC) placement system ("placement system") 10 generally including a needle 20, a guidewire 30, a syringe system 40, and a RICC catheter 50. The RICC catheter 50 can generally include a catheter 52 supported at a proximal end by a catheter hub ("hub") 60. The hub 60 can include one or more extension legs 62 extending proximally therefrom. Each extension leg 62 of the one or more extension legs can be in fluid communication with a lumen of the catheter 52. The catheter 52 can include a first section 54 disposed distally and defining a single lumen, a second section 56 disposed proximally and defining two or more lumen, and a dilator section 58 disposed therebetween. A guidewire 30 can extend through a lumen of the RICC catheter 50 from a proximal end of an extension leg 62, to a distal tip of the first section 54.

In an exemplary method of placing the RICC catheter 50, the needle 20 can be urged distally into the patient and access a vasculature, forming an insertion site. A syringe system 40, or similar device can draw a fluid flow proximally through a needle lumen 22 to observe a color and/or pulsatile flow and confirm correct vascular access. Once correct vascular access has been confirmed, the guidewire 30 can then be advanced through the needle lumen 22 and into the vasculature to maintain patency of the insertion site. The needle 20 and syringe system 40 assembly can then be withdrawn proximally. In an embodiment, a distal tip of the guidewire 30 can reside within the needle lumen 22 during venipuncture, which can expedite accessing the vasculature once venous access is confirmed and maintain patency of the insertion site. In an embodiment, the needle 20 can include a slot 26 configured to facilitate removal of the needle 20 and syringe system 40 from the guidewire 30 while leaving the guidewire 30 in place, as described in more detail herein.

The RICC 50 can then be advanced over the guidewire 30 and into the vasculature. The first section 54 of the RICC 50, having only a single lumen and defining a relatively smaller outer diameter, can enter the vasculature over the guidewire 30. The dilator section 58 can then dilate the insertion site to allow the relatively larger diameter second section 56, defining two or more lumen, to enter the vasculature. Once the RICC 50 has been placed, the guidewire 30 can be withdrawn proximally. Further details and embodiments of RICC systems 10 can be found, for example, in U.S. 10,376,675, U.S. 2019/0255294, U.S. 2021/0069471, U.S. 2021/0085927, U.S. 2021/0113809, U.S. 2021/0113810, U.S. 2021/0121661, U.S. 2021/0228843, U.S. 2021/0283368, U.S. 2021/0283381, U.S. 2021/0322729, U.S. 2021/0330941, U.S. 2021/0330942, U.S. 2021/0361915, U.S. 2021/0379336, U.S. 2021/0402142, U.S. 2021/0402149, U.S. 2021/0402153, U.S. 2021/0121667, U.S. 2022/0001138, U.S. 2022/0032013, U.S. 2022/0032014, U.S. 2022/0062528, U.S. 2022/0126064, U.S. 2022/0152368, U.S. 2022/0176081, U.S. 2022/0176082, U.S. 2022/0193376, U.S. 2022/0193377, U.S. 2022/0193378, U.S. 2022/0193379, and U.S. 2022/0296862.

FIGS. 1B-1C show further details of the slotted needle 20 of the RICC system 10. The needle 20 can define a lumen 22 and can be supported at a proximal end by a needle hub 28. The needle hub 28 can be coupled to the syringe system 40 and can provide fluid communication between the syringe system 40 and the needle lumen 22. In an embodiment, the needle 20 can include a guidewire aperture 24 disposed in a side wall of the needle 20, adjacent the needle hub 28 and communicating with the needle lumen 22. The guidewire aperture 24 can be configured to receive a portion of the guidewire 30 extending therethrough and into to the needle lumen 22. In an embodiment, a distal tip of the guidewire 30 can be disposed within the needle lumen 22 as the needle 20 accesses the vasculature. Once the needle 20 has accessed the vasculature, the distal tip of the guidewire 30 can extend distally of a distal tip of the needle 20. Advantageously, this allows for rapid stabilization of the insertion site soon after venipuncture, securing the insertion site and expediting the placement process.

In an embodiment, the needle 20 can further include a needle slot 26 extending longitudinally between the guidewire aperture 24, and a distal tip of the needle 20. In an embodiment, a lateral width of the needle slot 26 can be equal to or greater than a diameter of the guidewire 30. As such, a portion of the guidewire 30 can pass through the needle slot 26 to allow the needle 20 to disengage the guidewire 30. In an embodiment, a lateral width of the needle slot 26 can be equal to or less than a diameter of the guidewire 30. In an embodiment, a first edge of the needle slot 26 can contact a second edge of the needle slot 26, opposite the first edge across a central longitudinal axis to define a slit. In an embodiment, the first edge and the second edge of the needle slot 26 can be configured to flex laterally, or radially, outward and allow a portion of the guidewire 30 to pass through the needle slot 26 to allow the needle 20 to disengage the guidewire 30.

In an embodiment, the needle 20 can further include a sheath 70 disposed on an outer surface the needle 20. In an embodiment, the sheath 70 can be formed of a plastic, polymer, or similar suitable material. The sheath 70 can fit tightly about the needle 20, over the slot 26 and prevent any fluid leaking from the slot 26 to maintain the integrity of the needle lumen 22. In an embodiment, the sheath 70 can include a sheath guidewire aperture 74 disposed in a side wall of the sheath 70, adjacent a proximal end of the sheath 70 and aligned with the needle guidewire aperture 24 to communicate with the needle lumen 22. The sheath guidewire aperture 74 can be configured to receive a portion of the guidewire 30 extending therethrough and into to the needle lumen 22.

In an embodiment, the sheath 70 can include a tear line 72, extending longitudinally between the sheath guidewire aperture 74 and a distal end of the sheath 70. The tear line 72 can include a groove, score line, perforation, laser cut line, or similar line of weakness configured to allow the sheath 70 to separate therealong as the guidewire 30 is urged through the needle slot 26. In an embodiment, the placement system 10 can further include a blade configured to cut the sheath 70 along the tear line 72 to facilitate disengaging the guidewire 30 from the needle 20.

In an exemplary method of use, the needle 20 and sheath 70 assembly can be urged distally to form an insertion site as described herein. A fluid flow can flow proximally through the needle 22. The sheath 70 disposed over the slot 26 can prevent fluid from leaking from the lumen 22 through the slot 26. In an embodiment, a vacuum can be applied to the needle lumen 22, for example by the syringe system 40, to draw a fluid flow therethrough. Advantageously, the sheath 70 can maintain the integrity of the needle lumen 22 to prevent fluid being drawn through the slot 26, and instead draw a fluid through a distal opening of the needle lumen, proximate the distal tip.

Once vascular access has been confirmed, the needle 20 can be withdrawn proximally. To allow the guidewire 30 to remain in position as the needle 20 is withdrawn proximally, a portion of the guidewire 30 can pass through the slot 26 from the guidewire aperture 24 to a distal end of the needle 20. As the guidewire 30 passes through the slot 26, the guidewire 30 can tear the sheath 70 along the tear line 72 allowing the guidewire 30 to separate from the needle 20 and sheath 70 assembly. As will be appreciated, various apparatus and methods of removing the needle 20 while leaving the guidewire 30 in place within the vasculature are contemplated to fall within the scope of the present invention. Further details and embodiments of such systems can be found in U.S. Patent Application No. 17/746,113 filed May 17, 2022, and U.S. Patent Application No. 17/883,490 filed August 8, 2022.

In an embodiment, the RICC placement system 10 can include a guidewire locking system 100 configured to facilitate stabilization of the guidewire 30 relative to the insertion site, as the needle 20 is withdrawn proximally. Advantageously, the guidewire locking system 100 can mitigate any movement of the guidewire 30 within the vasculature, or prevent inadvertent removal of the guidewire 30 from the vasculature.

In an embodiment, as shown in FIG. 2A, the guidewire locking system 100 can generally include a housing 110 defining a needle channel 112 and a guidewire channel 114. The guidewire channel 114 can communicate with the needle channel 112 and extend at an angle therefrom. The needle channel 112 can be configured to receive a portion of the needle 20 therethrough. When the needle 20 is engaged with the housing 110, the guidewire aperture 24 of the needle 20 can align with the guidewire channel 114. As such, the guidewire 30 can extend through the guidewire channel 114, through the guidewire aperture 24 of the needle 20 and into the needle lumen 22. In an embodiment, a proximal end of the housing 110 can releasably engage the needle hub 28 in an interference fit, press fit, snap fit engagement, luer lock, threaded engagement, combinations thereof, or the like. A proximal end of the needle hub 28 can releasably engage a syringe system 40 in a press-fit, interference fit, snap-fit, luer lock, threaded engagement, combinations thereof, or the like.

The housing 110 can further include one or more handles 118 configured to allow a user to grasp and manipulate the housing 110. For example, a user can grasp the handle 118 and stabilize guidewire locking system 100 relative to the insertion site, while the user urges the needle hub 28 proximally to disengage the needle hub 28 from the housing 110 and withdraw the needle 20 proximally from the needle channel 112. In an embodiment, the guidewire locking system 100 can further include a sealing member ("seal") 120 disposed within one or both of the needle channel 112 and the guidewire channel 114. The seal 120 can be formed of a compliant material, including a plastic, polymer, elastomer, silicone, rubber, combinations thereof, or the like.

In an embodiment, the seal 120 can define a seal needle lumen 122 configured to receive a portion of the needle 20 therethrough, and a seal guidewire lumen 124 configured to receive the guidewire 30 therethrough. In an embodiment, a diameter of the seal needle lumen 122 can be equal to, or less than, an outer diameter of the needle 20. As such, the seal 120 can slidably engage the needle 20 in an interference fit while preventing a fluid flow between the outer surface of the needle 20 and an inner surface of the seal needle lumen 122. Similarly, a diameter of the seal guidewire lumen 124 can be equal to, or less than, an outer diameter of the guidewire 30. As such, the seal 120 can slidably engage the guidewire 30 in an interference fit. Worded differently, a user can slide the guidewire 30 through the seal lumen 124 to a first position, and the seal 120 can engage and retain the guidewire 30 in the first position until the guidewire 30 is repositioned to a second position. In an embodiment, the seal 120 can fit tightly about one or both of the needle 20 and the guidewire 30 to provide a fluid tight seal between the housing 110 and one or both of the needle 20 and the guidewire 30. For example, when a vacuum is applied to the needle lumen 22 by the syringe system 40, the seal 120 can prevent leakage of fluids, e.g. to/from the guidewire aperture 24, or the like.

In an embodiment one or more of the seal 120, guidewire 30, and/or needle 20 can include a biocompatible, lubricious coating disposed on a surface thereof to provide a fluid tight seal therebetween. Exemplary lubricious coatings can include hydrogels, silicone oil, silicone grease, or similar suitable lubricants. Advantageously, the sealing member 120 can form a robust seal between one or both of the needle 20 and the guidewire 30 and the housing 110 to support a vacuum pressure during blood flash visualization.

In an embodiment, the guidewire locking system 100 can further include a locking mechanism 130 configured to lock the guidewire 30 relative to the housing 110, preventing any movement therebetween. As shown in FIG. 2A, the locking mechanism 130 can include a "Tuohy-Borst" style locking mechanism 130 defining a lock lumen 132 configured to align with the seal guidewire lumen 124 and receive a portion of the guidewire 30 therethrough. In an embodiment, a proximal end of the lock lumen 132 can define a funnel shape, configured to facilitate directing a distal tip of the guidewire 30 into the lock lumen 132.

In an embodiment, a distal end of the locking mechanism 130 can define a tapered recess 134 configured to receive a portion of the seal 120 therein. In use, as the locking mechanism 130 is urged distally into the guidewire channel 114, the tapered recess 134 can compress a portion of the seal 120 radially inwards, onto the guidewire 30 gripping the guidewire 30 and preventing the guidewire 30 from sliding relative to the housing 110.

In an embodiment, the locking mechanism 130 can be threadably engaged with the housing 110. For example, an inner surface of a collar 138 of the locking mechanism 130 can threadably engage an outer surface of the housing 110. However, it will be appreciated that other configurations of threaded engagement are also contemplated to fall within the scope of the present invention. Rotating the collar 138 can rotate the locking mechanism about the axis of the guidewire 30 and urge the tapered recess 134 onto the seal 120 to grip the guidewire 30.

In an exemplary method of use, a user can rotate the collar 138 about an axis of the guidewire channel 114 to threadably engage the housing 110 and urge a lock recess 134 distally. The lock recess 134 can compress the seal 120 radially inwards, gripping the guidewire 30 and preventing longitudinal movement relative to the housing 110. Similarly, a user can rotate the collar 138 in an opposite direction to retract the locking mechanism 130 from the seal 120 and allow the guidewire 30 to slide relative to the housing 110.

In an embodiment, the seal 120 can further include a seal slot 126 extending longitudinally along a top surface of the seal and communicating with one or both of the seal needle lumen 122 and the seal guidewire lumen 124. The seal slot 126 can be configured to allow the guidewire 30 to pass therethrough and allow the guidewire locking system 100 to disengage the guidewire 30. In an embodiment, when the housing 110 is engaged with the needle hub 28, one or both of the needle hub 28 and the housing 110 can apply a laterally inward pressure on the seal 120, compressing opposing portions of the seal slot 26 together and forming a fluid tight seal therebetween. When the housing 110 is disengaged from the needle hub 28, the laterally inward pressure is released, allowing the guidewire 30 to pass through the seal slot 28.

In an embodiment, the guidewire locking system 100 can further include a peel pin 128 extending laterally through the seal 120 and disposed adjacent a junction between the seal needle lumen 122 and the seal guidewire lumen 124. In an embodiment, a cross-sectional shape of the peel pin 128 can define a circular, oval, ellipsoidal, wedge, or triangular shape. However it will be appreciated that other cross-sectional shapes are also contemplated. In an embodiment, the peel pin 128 can be formed integrally with the housing 110. In an embodiment, the peel pin 128 can be formed as a separate structure from the housing and coupled thereto with an interference fit, press-fit, snap-fit engagement, adhesive, bonding, welding, combinations thereof, or the like.

In an embodiment, the peel pin 128 can provide a structural support to the guidewire 30 at the point where the guidewire 30 disengages the needle 20. The peel pin 128 can prevent the needle slot 26 from "dragging" the guidewire 30 proximally, as the needle 20 and guidewire 30 as withdrawn and disengaged. This can prevent the guidewire 30 from kinking or snagging on the needle slot 26 preventing disengagement and failure of the guidewire locking system 100.

In an embodiment, as shown in FIGS. 2B-2C, the housing 110 can include a housing slot 116 extending longitudinally between a proximal end of the guide channel 114 and a distal end of the needle channel 112, and communicating with one or both of the guide channel 114 and the needle channel 112. In an embodiment, the locking mechanism 130 can include a lock slot 136 extending longitudinally between the distal end and the proximal end of the locking mechanism 130 and communicating with the lock lumen 132. Further the seal 120 can include a seal slot 126, as described herein.

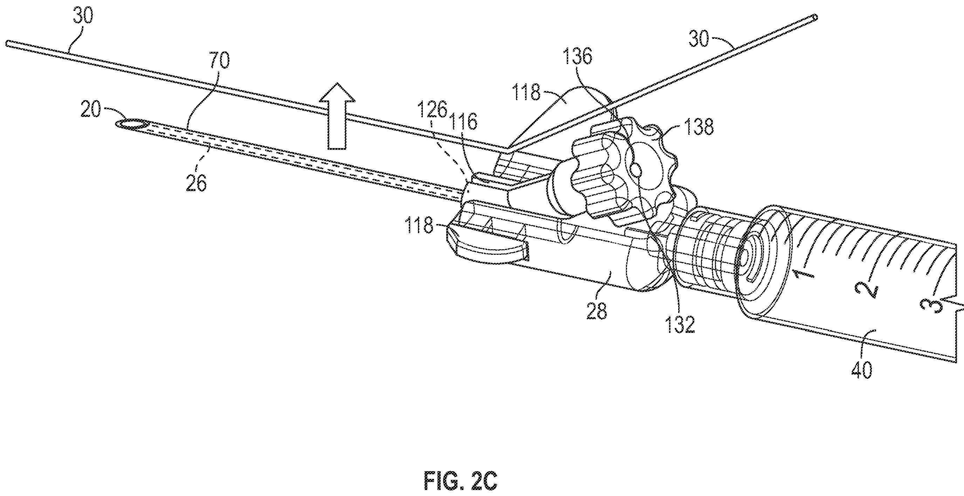

In an embodiment, with the locking mechanism 130 in the locked position (FIG. 2B), the lock slot 136 can be mis-aligned with the housing slot 116 preventing the guidewire 30 from passing through seal slot 126, housing slot 116 and/or the lock slot 136. With the locking mechanism 130 in the unlocked position (FIG. 2C), the lock slot 136 can align with the housing slot 116. As such, the guidewire 130 can pass through the seal slot 126, housing slot 116 and the lock slot 136 to allow the housing 110 to disengage the guidewire 30 along an axis extending at an angle to the longitudinal axis, e.g. a transverse axis.

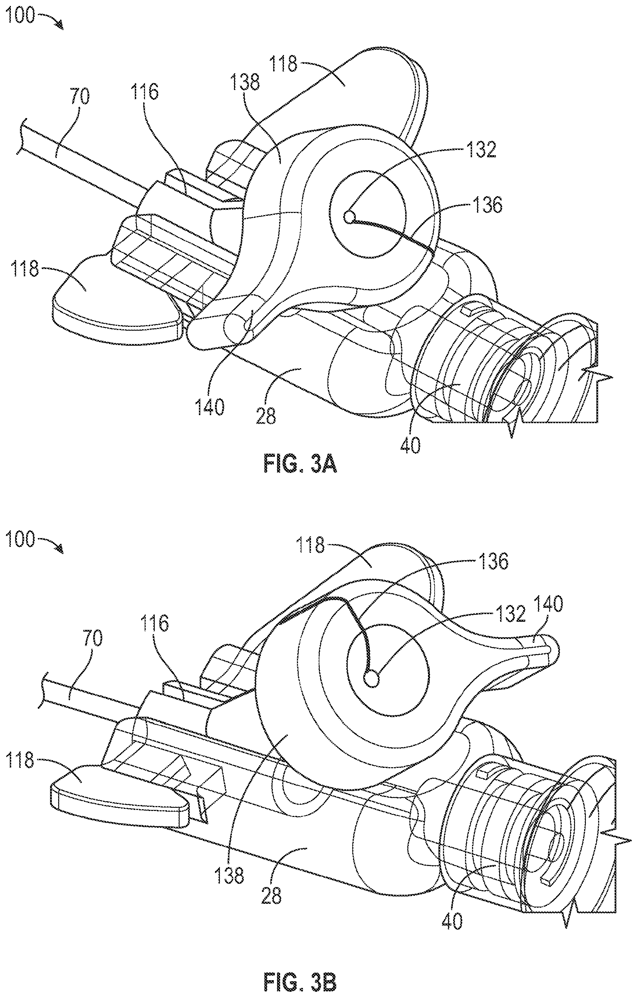

In an embodiment, as shown in FIGS. 3A-3B, the collar 138 can include a lever 140 extending therefrom, perpendicular to an axis of the lock lumen 132. Advantageously, the lever 140 can provide mechanical advantage to facilitate rotating the collar 138 about the axis of the lock lumen 132, providing a secure engagement with the guidewire 30, as described herein. Further, a position of the lever 140 relative to the housing 110 can provide a quick and intuitive indicator to the user as to whether the guidewire locking system 100 is in the locked or unlocked position. In an embodiment, one or more of the collar 138, lever 140, housing 110, combinations thereof, or the like can include a symbol, different colored portions, alphanumeric symbol, or the like to indicated to a user when the collar 138 is in one of the locked or unlocked positions. For example, a first side of the lever 140 can include an open padlock symbol, green color, or the like to indicate the collar 138 is in the unlocked position, and a second side of the lever 140 can include a closed padlock symbol, red color, or the like to indicate the collar 138 is in the locked position. These and other combinations of indicators and associated mechanisms are contemplated to fall within the scope of the present invention.

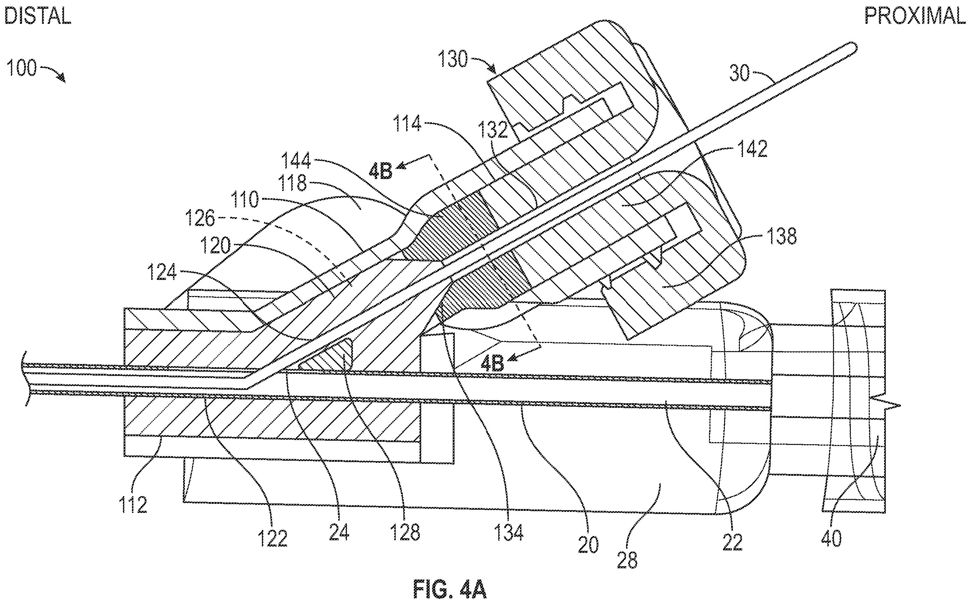

In an embodiment, as shown in FIGS. 4A-4B the locking mechanism 130 can include a rotation piece 142 and a compression piece 144. In an embodiment, the rotation piece 142 and the compression piece 144 can co-operate to define the lock lumen 132. A proximal end of the compression piece 144 can be rotatably coupled to a distal end of the rotation piece 142. As such, the rotation piece 142 can rotate freely relative to the position of the compression piece 144, about the axis of the lock lumen 132. In an embodiment, the proximal end of the rotation piece 142 can be fixedly coupled to the collar 138. As such, rotating the collar 138 can rotate the rotation piece 142 about the axis of the lock lumen 132. Further the compression piece 144 can remain rotationally stationary relative to the rotation piece 142 as the rotation piece 142 and the collar 138 are rotated.

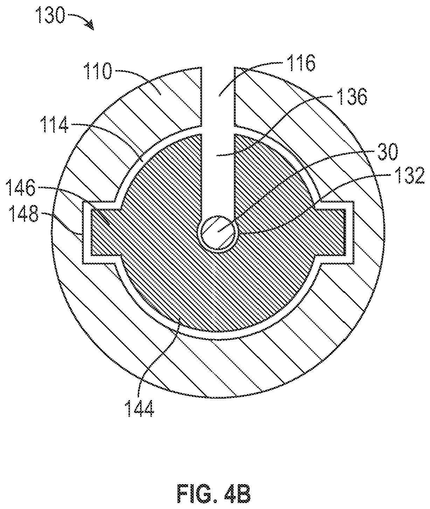

In an embodiment, as shown in FIG. 4B, the rotation piece 144 can include a rail 146 extending from an outer surface of the rotation piece 144 and extending parallel with the axis of the lock lumen 132. The rail 146 can be configured to engage a recess 148 disposed in the inner wall of the guidewire channel 114 of the housing 110 and also extending parallel with an axis of the lock lumen 132. In an embodiment, the rail 146 can engage the recess 148 to prevent rotational movement of the compression piece 144 about the axis of the lock lumen 132. Further, the rail 146 can allow the compression piece 146 to slide relative to the housing along the axis of the lock lumen 132.

Advantageously, the compression piece 144 can prevent the locking mechanism 130 from applying any rotational movement to the seal 120, while also applying a longitudinal force to the seal 120, as the locking mechanism 130 is rotated and transitioned to the locked position. As such, the locking mechanism 130 can prevent any twisting of the seal 120 about the guidewire 30 which might misalign the seal slot 126 with the housing slot 116, preventing disengagement of the guidewire 30 from the guidewire locking system 100. Further, any rotational movement applied to the seal 120 may cause a crease or the like, causing failure of the seal 120. As such, the compression piece 144 can remain rotationally stationary relative to the rotation piece 142 to mitigate creasing or failure of the seal 120.

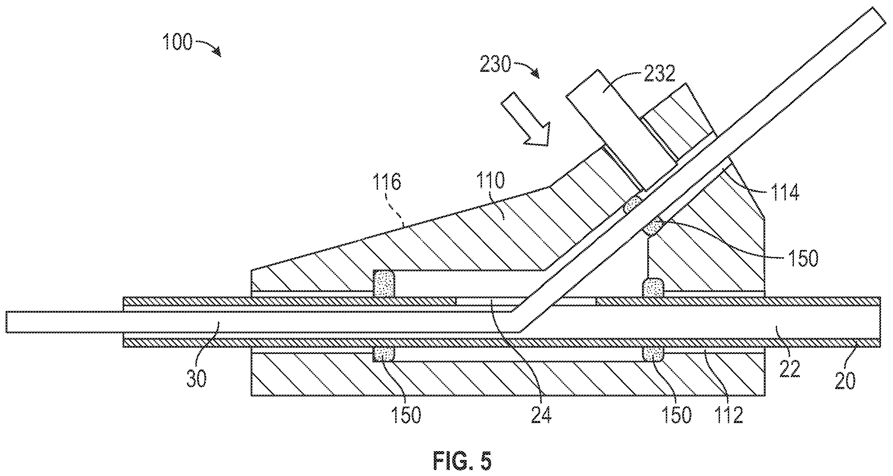

In an embodiment, as shown in FIG. 5, the guidewire locking system 100 can include a non-axial, or perpendicular locking mechanism 230. The perpendicular locking mechanism 230 can include an actuator 232 slidably engaged with the housing 110 along an axis extending at an angle, e.g. perpendicular, relative to an axis of the guidewire channel 114. In an embodiment, the actuator 232 can transition between an unlocked position (FIG. 5) and a locked position. In the unlocked position the guidewire 30 can slide along the guidewire channel 114 with little or no resistance. In the locked position, a surface of the actuator 232 can impinge a portion of the guidewire 30 against an inner surface of the guidewire channel 114 to prevent the guidewire 30 from sliding relative to the housing 110.

In an embodiment, the actuator 232 can engage the housing in a push-button mechanism wherein a first axial force applied to the actuator 232 can transition the actuator 232 from the unlocked position to the locked position. A second axial force applied to the actuator 232 can transition the actuator 232 from the locked position to the unlocked position. In an embodiment, the first axial force and the second axial force can be applied along the same axis and/or in the same direction. In an embodiment, the first axial force and the second axial force can be applied along different axes or along different directions, e.g. opposite directions. In an embodiment, the actuator 232 can be threadably engaged with the housing 110 and can include a rotation piece and a compression piece, as described herein. As such, rotating the rotation piece can urge the compression piece along an axis perpendicular to the axis of the guidewire 30 until the compression piece impinges on the guidewire 30. As will be appreciated these and other combinations of non-axial, perpendicular locking mechanisms 230 including levers, cam mechanisms or the like, that cause the actuator to engage the guidewire 30 at an angle relative to a guidewire axis, are also contemplated to fall within the scope of the present invention. Advantageously, the perpendicular locking mechanism 230 can apply a force extending parallel to an axis of the housing slot 116, and can mitigate applying a force which may cause misalignment the guidewire 30 with the housing slot 116.

In an embodiment, the guidewire locking system 100 can include one or more valves 150 configured to allow one or both of the needle 20 and the guidewire 30 to slide axially while controlling a fluid flow through one or both of the needle channel 112 and the guidewire channel 114 of the housing 110. Advantageously, the one or more valves 150 can mitigate a fluid leakage from the guidewire aperture 24 as a fluid passes through the needle lumen 22, or mitigate a fluid leakage into the guidewire aperture 24 when a vacuum is applied to the needle lumen 22, as described herein. Further, the one or more valves 150 can engage one or both of the needle 20 and the guidewire 30 in an interference fit to prevent one or both of the needle 20 and the guidewire 30 from sliding freely. Worded differently, with the locking mechanism 130, or the perpendicular locking mechanism 230, are in the unlocked position a user can position one or both of the needle 20 and guidewire 30 in a first position relative to the housing 110 and the one or more valves 150 can engage the needle 20 and/or guidewire 30 and can remain in the first position until repositioned to a second position.

In an exemplary method of use a RICC system 10 is provided including a guidewire locking system 100, as described herein. In an embodiment, a distal tip of the guidewire 30 extends into the lumen of the needle 20. The RICC system 10 is urged distally to access a vasculature of a patient with the needle 20 and confirm vascular access, as described herein. Once vascular access is confirmed, the guidewire 30 can be advanced through the needle lumen 22 until a distal tip of the guidewire 30 extends distally of the distal tip of the needle 22 and into the vasculature. Optionally, the user can transition the locking mechanism 130, 230 to the unlocked position prior to advancing the guidewire 30 distally through the needle lumen 22 and into the vasculature of the needle 22.

Once the guidewire 30 has been positioned, the user can then transition the locking mechanism 130, 230 from the unlocked position to the locked position. In an embodiment, this includes rotating a collar 138 or a lever 140. In an embodiment, this includes rotating a rotation piece 142 and advancing a compression piece 142 onto a seal 120. In an embodiment, this includes compressing the seal 120 radially inwards to impinge on the guidewire 30 and prevent axial movement of the guidewire 30 relative to the housing 110. In an embodiment, a user can actuate an actuator 232 along an axis extending at an angle relative to the axis of the guidewire 30 to impinge on the guidewire 30 and prevent axial movement thereof.

The user can grasp the handle 118 and stabilize the housing 110 relative to the insertion site. The user can then detach the needle hub 28 from the housing 110 and urge the needle 20 proximally through the needle channel 112 of the housing 110. As the needle 20 slides proximally, a portion of the guidewire 30, supported by the peel pin 128 can be urged from the guidewire aperture 24, through the needle slot 26 to disengage the needle 20 from the guidewire 30. In an embodiment, the guidewire 30 can tear the sheath 70 along the tear line 72.

In an embodiment, once the needle 20 has been removed from the housing 110, the locking mechanism 130, 230 can be transitioned to the unlocked position. In the unlocked position one or both of the seal slot 126 and the lock slot 136 can align with the housing slot 116 and can allow the guidewire 30 to pass therethrough. This can allow the housing 110 to disengage the guidewire 30. The RICC catheter 50 can then be urged over the guidewire 30 and into the vasculature of the patient. Once the RICC catheter 50 has been placed, the guidewire 30 can be withdraw proximally from the lumen of the RICC catheter 50.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A catheter placement system (10), comprising:
a needle (20) supported by a needle hub (28) and defining a needle lumen (22);
a guidewire (30) having a distal tip disposed within the needle lumen (22);
a catheter (52) disposed on a proximal portion of the guidewire (30); and
a guidewire locking system (100), comprising:
a housing (100) defining a needle channel (112) and a guidewire channel (114) communicating with the needle channel (112) and extending at an angle thereto, a portion of the needle (20) extending through the needle channel (112) and a portion of the guidewire (30) extending through the guidewire channel (114); and
a locking mechanism (130) having a lock and a seal (120) and transitionable between an unlocked position and a locked position, the locking mechanism (130) in the locked position configured to inhibit axial movement of the guidewire (130) through the guidewire channel (114).

2. The catheter placement system according to claim 1, wherein the seal (120) is formed of a compliant material and defines a seal guidewire lumen (124) configured to receive a portion of the guidewire (30) therethrough, the lock in the locked position configured to compress the seal (120) radially inwards to impinge on the guidewire (30) and inhibit axial movement thereof.

3. The catheter placement system according to claim 2, wherein a diameter of the seal guidewire lumen (124) is equal to or less than an outer diameter of the guidewire (30) and engages the guidewire in an interference fit in one or both of the locked position and the unlocked position.

4. The catheter placement system according to any of the preceding claims, wherein the lock includes a tapered distal end (134) configured to receive a portion to the seal (120) therein and compress the seal radially inwards as the locking mechanism (130) is transitioned to the locked position.

5. The catheter placement system according to any of the preceding claims, wherein the lock includes a collar (138) threadably engaged with the housing (100) and configured to urge the lock axially through the guidewire channel (114) as the lock is rotated about the axis of the guidewire, optionally
wherein the collar (138) includes a lever (140) extending therefrom, perpendicular to an axis of the guidewire channel (114).

6. The catheter placement system according to any of the preceding claims, further including a peel pin (128) extending through the seal (120) and disposed at a junction between the guidewire channel (114) and the needle channel (112) and configured to support the guidewire (30) as the needle (20) is withdrawn proximally from the needle channel (112).

7. The catheter placement system according to claim 6, wherein the peel pin (128) is formed integrally with the housing (110), or wherein the peel pin (128) is formed as a separate structure and coupled with the housing (110) using one of an interference fit, press-fit, snap-fit engagement, adhesive, bonding, or welding.

8. The catheter placement system according to any of the preceding claims, wherein a proximal end of the housing (110) is releasably engaged with the needle hub (28) in one of an interference fit, press fit, or snap fit engagement.

9. The catheter placement system according to any of the preceding claims, further including a handle (118) extending from the housing (110) and configured to facilitate grasping and manipulating the housing relative to the needle hub (28).

10. The catheter placement system according to any of the preceding claims, wherein the housing (110) includes a housing slot (116) extending longitudinally and communicating with one or both of the needle channel (112) and the guidewire channel (114), the housing slot configured to allow the guidewire (30) to pass therethrough when the locking mechanism (130) is in the unlocked position.

11. The catheter placement system according to any of the preceding claims, wherein the seal (120) includes a seal slot (126) extending longitudinally and communicating with one or both of the seal guidewire lumen (124) and a seal needle lumen (122), the seal slot configured to allow the guidewire (30) to pass therethrough when the locking mechanism (130) is in the unlocked position.

12. The catheter placement system according to either claim 10 or claim 11, wherein the lock includes a lock slot (136) extending longitudinally and communicating with a lock lumen (132), the lock slot configured to align with the housing slot (116) in the unlocked position and allow the guidewire (30) to pass therethrough to disengage the guidewire locking system.

13. The catheter placement system according to any of the preceding claims, wherein the needle (20) includes a guidewire aperture (24) disposed in a side wall of the needle and configured to receive a portion of the guidewire (30) therethrough, the needle also including a needle slot (26) extending longitudinally between the guidewire aperture and a distal tip of the needle and communicating with a needle lumen (22), the needle slot configured to receive a portion of the guidewire (30) therethrough as the needle is withdrawn proximally, and/or
further including a sheath (70) encircling a portion of the needle (20) and including a tear line (72) extending longitudinally between a proximal end and a distal end of the sheath.

14. The catheter placement system according to any of the preceding claims, wherein the lock includes a compression piece (142) rotatably coupled to a distal end of a rotation piece (144), the rotation piece configured to rotate about an axis of the guidewire channel (114) and urge the compression piece axially, and/or
wherein the compression piece (142) includes a rail (146) extending from an outer surface thereof and configured to engage a recess (148) disposed in an inner wall of the guidewire channel (114) to mitigate rotational movement of the compression piece while allowing axial movement thereof.

15. The catheter placement system according to claim 1, wherein the locking mechanism (130) includes an actuator (232) slidably engaged with the housing (110) along an axis extending perpendicular to an axis of the guidewire channel (114), the actuator transitionable between the locked and unlocked position, optionally
wherein the locking mechanism (130) includes a push-button mechanism having a first force applied in a first direction to transition the actuator (232) from the unlocked position to the locked position and a second force also applied in the first direction to transition the actuator (232) from the locked position to the unlocked position.

## Patentansprüche

1. Katheterplatzierungssystem (10), umfassend:
eine Nadel (20), die durch eine Nadelnabe (28) gestützt wird und ein Nadellumen (22) definiert;
einen Führungsdraht (30), der eine distale Spitze aufweist, die innerhalb des Nadellumens (22) angeordnet ist;
einen Katheter (52), der an einem proximalen Abschnitt des Führungsdrahts (30) angeordnet ist; und
ein Führungsdrahtverriegelungssystem (100), umfassend:
ein Gehäuse (100), das einen Nadelkanal (112) und einen Führungsdrahtkanal (114) definiert, der mit dem Nadelkanal (112) kommuniziert und sich in einem Winkel dazu erstreckt, wobei sich ein Abschnitt der Nadel (20) durch den Nadelkanal (112) erstreckt und sich ein Abschnitt des Führungsdrahts (30) durch den Führungsdrahtkanal (114) erstreckt; und
einen Verriegelungsmechanismus (130), der eine Verriegelung und eine Dichtung (120) aufweist und zwischen einer entriegelten Position und einer verriegelten Position überführbar ist, wobei der Verriegelungsmechanismus (130) in der verriegelten Position dazu ausgebildet ist, axiale Bewegung des Führungsdrahts (130) durch den Führungsdrahtkanal (114) hindurch zu verhindern.

2. Katheterplatzierungssystem nach Anspruch 1, wobei die Dichtung (120) aus einem nachgiebigen Material gebildet ist und ein Dichtungsführungsdrahtlumen (124) bildet, das dazu ausgebildet ist, einen Abschnitt des Führungsdrahts (30) dort hindurch aufzunehmen, wobei die Verriegelung in der verriegelten Position dazu ausgebildet ist, die Dichtung (120) radial nach innen zu komprimieren, um auf den Führungsdraht (30) einzuwirken und axiale Bewegung davon zu verhindern.

3. Katheterplatzierungssystem nach Anspruch 2, wobei ein Durchmesser des Dichtungsführungsdrahtlumens (124) gleich oder kleiner als ein Außendurchmesser des Führungsdrahts (30) ist und in den Führungsdraht in einer Übermaßpassung in einer oder beiden von der verriegelten Position und der entriegelten Position eingreift.

4. Katheterplatzierungssystem nach einem der vorstehenden Ansprüche, wobei die Verriegelung ein verjüngtes distales Ende (134) einschließt, das dazu ausgebildet ist, einen Abschnitt der Dichtung (120) darin aufzunehmen und die Dichtung radial nach innen zu komprimieren, wenn der Verriegelungsmechanismus (130) in die verriegelte Position überführt wird.

5. Katheterplatzierungssystem nach einem der vorstehenden Ansprüche, wobei die Verriegelung einen Kragen (138) einschließt, der mit dem Gehäuse (100) in Gewindeingriff steht und dazu ausgebildet ist, die Verriegelung axial durch den Führungsdrahtkanal (114) hindurchzuzwängen, wenn die Verriegelung um die Achse des Führungsdrahtes herum gedreht wird, wobei optional
der Kragen (138) einen Hebel (140) einschließt, der sich davon senkrecht zu einer Achse des Führungsdrahtkanals (114) erstreckt.

6. Katheterplatzierungssystem nach einem der vorstehenden Ansprüche, weiter einschließend einen Abziehstift (128), der sich durch die Dichtung (120) hindurch erstreckt und an einer Verbindungsstelle zwischen dem Führungsdrahtkanal (114) und dem Nadelkanal (112) angeordnet ist und dazu ausgebildet ist, den Führungsdraht (30) zu stützen, wenn die Nadel (20) proximal aus dem Nadelkanal (112) herausgezogen wird.

7. Katheterplatzierungssystem nach Anspruch 6, wobei der Abziehstift (128) einstückig mit dem Gehäuse (110) gebildet ist oder wobei der Abziehstift (128) als eine separate Struktur gebildet ist und mit dem Gehäuse (110) unter Verwendung von einem von einem Übermaßpassungs-, Presspassungs- , Rastpassungseingriff, Klebstoff, Bonding oder Schweißen gekoppelt ist.

8. Katheterplatzierungssystem nach einem der vorstehenden Ansprüche, wobei ein proximales Ende des Gehäuses (110) lösbar mit der Nadelnabe (28) durch Übermaßpassungs-, Presspassungs- oder Rastpassungseingriff in Eingriff steht.

9. Katheterplatzierungssystem nach einem der vorstehenden Ansprüche, weiter einschließend einen Griff (118), der sich von dem Gehäuse (110) erstreckt und dazu ausgebildet ist, Ergreifen und Handhaben des Gehäuses relativ zu der Nadelnabe (28) zu erleichtern.

10. Katheterplatzierungssystem nach einem der vorstehenden Ansprüche, wobei das Gehäuse (110) einen Gehäuseschlitz (116) einschließt, der sich längs erstreckt und mit einem oder beidem von dem Nadelkanal (112) und dem Führungsdrahtkanal (114) in Kommunikation steht, wobei der Gehäuseschlitz dazu ausgebildet ist, zu ermöglichen, dass der Führungsdraht (30) dort hindurchgeht, wenn sich der Verriegelungsmechanismus (130) in der entriegelten Position befindet.

11. Katheterplatzierungssystem nach einem der vorstehenden Ansprüche, wobei die Dichtung (120) einen Dichtungsschlitz (126) einschließt, der sich längs erstreckt und mit einem oder beidem von dem Dichtungsführungsdrahtlumen (124) und dem Dichtungsnadellumen (122) in Kommunikation steht, wobei der Dichtungsschlitz dazu ausgebildet ist, zu ermöglichen, dass der Führungsdraht (30) dort hindurchgeht, wenn sich der Verriegelungsmechanismus (130) in der entriegelten Position befindet.

12. Katheterplatzierungssystem nach Anspruch 10 oder Anspruch 11, wobei die Verriegelung einen Verriegelungsschlitz (136) einschließt, der sich längs erstreckt und mit einem Verriegelungslumen (132) in Kommunikation steht, wobei der Verriegelungsschlitz dazu ausgebildet ist, sich in der entriegelten Position mit dem Gehäuseschlitz (116) auszurichten und zu ermöglichen, dass der Führungsdraht (30) dort hindurchgeht, um das Führungsdrahtverriegelungssystem außer Eingriff zu bringen.

13. Katheterplatzierungssystem nach einem der vorstehenden Ansprüche, wobei die Nadel (20) eine Führungsdrahtapertur (24) einschließt, die in einer Seitenwand der Nadel angeordnet ist und dazu ausgebildet ist, einen Abschnitt des Führungsdrahts (30) dort hindurch aufzunehmen, wobei die Nadel zudem einen Nadelschlitz (26) einschließt, der sich längs zwischen der Führungsdrahtapertur und einer distalen Spitze der Nadel erstreckt und mit einem Nadelumen (22) in Kommunikation steht, wobei der Nadelschlitz dazu ausgebildet ist, einen Abschnitt des Führungsdrahts (30) dort hindurch aufzunehmen, wenn die Nadel proximal zurückgezogen wird, und/oder
weiter einschließend eine Hülle (70), die einen Abschnitt der Nadel (20) umgibt und eine Aufreißlinie (72) einschließt, die sich längs zwischen einem proximalen Ende und einem distalen Ende der Hülle erstreckt.

14. Katheterplatzierungssystem nach einem der vorstehenden Ansprüche, wobei die Verriegelung ein Kompressionsstück (142) einschließt, das drehbar an ein distales Ende eines Drehstücks (144) gekoppelt ist, wobei das Drehstück dazu ausgebildet ist, sich um eine Achse des Führungsdrahtkanals (114) zu drehen und das Kompressionsstück axial zu zwängen, und/oder
wobei das Kompressionsstück (142) eine Schiene (146) einschließt, die sich von einer Außenfläche davon erstreckt und dazu ausgebildet ist, in eine in einer Innenwand des Führungsdrahtkanals (114) angeordnete Aussparung (148) einzugreifen, um eine Drehbewegung des Kompressionsstücks abzuschwächen, während sie gleichzeitig eine axiale Bewegung davon ermöglicht.

15. Katheterplatzierungssystem nach Anspruch 1, wobei der Verriegelungsmechanismus (130) einen Aktor (232) einschließt, der entlang einer Achse, die sich senkrecht zu einer Achse des Führungsdrahtkanals (114) erstreckt, verschiebbar mit dem Gehäuse (110) in Eingriff steht, wobei der Aktor zwischen der verriegelten und der entriegelten Position überführbar ist, wobei optional
der Verriegelungsmechanismus (130) einen Druckknopfmechanismus einschließt, der eine erste Kraft, die in einer ersten Richtung ausgeübt wird, um den Aktor (232) aus der entriegelten Position in die verriegelte Position zu bewegen, und eine zweite Kraft, die ebenfalls in der ersten Richtung ausgeübt wird, um den Aktor (232) aus der verriegelten Position in die entriegelte Position zu bewegen, aufweist.

## Revendications

1. Système (10) de placement de cathéter, comprenant :
une aiguille (20) supportée par un pavillon d'aiguille (28) et définissant une lumière d'aiguille (22) ;
un fil-guide (30) présentant une pointe distale disposée à l'intérieur de la lumière d'aiguille (22) ;
un cathéter (52) disposé sur une portion proximale du fil-guide (30) ; et
un système de verrouillage (100) de fil-guide, comprenant :
un logement (100) définissant un canal d'aiguille (112) et un canal de fil-guide (114) communiquant avec le canal d'aiguille (112) et s'étendant selon un angle par rapport à celui-ci, une portion de l'aiguille (20) s'étendant à travers le canal d'aiguille (112) et une portion du fil-guide (30) s'étendant à travers le canal de fil-guide (114) ; et
un mécanisme de verrouillage (130) présentant un verrou et un joint (120) et pouvant passer entre une position non bloquée et une position bloquée, le mécanisme de verrouillage (130) dans la position bloquée étant configuré pour inhiber un mouvement axial du fil-guide (130) à travers le canal de fil-guide (114).

2. Système de placement de cathéter selon la revendication 1, dans lequel le joint (120) est formé d'un matériau souple et définit une lumière (124) de fil-guide de joint configurée pour recevoir une portion du fil-guide (30) à travers celui-ci, le verrou dans la position bloquée étant configuré pour compresser le joint (120) radialement vers l'intérieur afin qu'il vienne en appui contre le fil-guide (30) et inhibe un mouvement axial de celui-ci.

3. Système de placement de cathéter selon la revendication 2, dans lequel un diamètre de la lumière (124) de fil-guide de joint est inférieur ou égal à un diamètre externe du fil-guide (30) et vient en prise avec le fil-guide selon un ajustement serré dans l'une ou les deux parmi la position bloquée et la position non bloquée.

4. Système de placement de cathéter selon l'une quelconque des revendications précédentes, dans lequel le verrou inclut une extrémité distale effilée (134) configurée pour recevoir une portion du joint (120) à l'intérieur de celle-ci et compresser le joint radialement vers l'intérieur lorsque le mécanisme de verrouillage (130) passe en position bloquée.

5. Système de placement de cathéter selon l'une quelconque des revendications précédentes, dans lequel le verrou inclut un collier (138) qui vient en prise par filetage avec le logement (100) et est configuré pour pousser le verrou axialement à travers le canal de fil-guide (114) lorsque le verrou est tourné autour de l'axe du fil-guide, facultativement
dans lequel le collier (138) inclut un levier (140) s'étendant à partir de celui-ci, perpendiculaire à un axe du canal de fil-guide (114).

6. Système de placement de cathéter selon l'une quelconque des revendications précédentes, incluant en outre une goupille de décollement (128) s'étendant à travers le joint (120) et disposée à une jonction entre le canal de fil-guide (114) et le canal d'aiguille (112) et configurée pour supporter le fil-guide (30) lorsque l'aiguille (20) est retirée de manière proximale du canal d'aiguille (112).

7. Système de placement de cathéter selon la revendication 6, dans lequel la goupille de décollement (128) est formée d'une seule pièce avec le logement (110), ou dans lequel la goupille de décollement (128) est réalisée sous la forme d'une structure séparée et couplée au logement (110) en utilisant l'un parmi un ajustement serré, un emmanchement à force, un engagement par encliquetage, un adhésif, un collage ou un soudage.

8. Système de placement de cathéter selon l'une quelconque des revendications précédentes, dans lequel une extrémité proximale du logement (110) est en prise de manière amovible avec le pavillon d'aiguille (28) selon l'un parmi un ajustement serré, un emmanchement à force, ou un engagement par encliquetage

9. Système de placement de cathéter selon l'une quelconque des revendications précédentes, incluant en outre une poignée (118) s'étendant à partir du logement (110) et configurée pour faciliter la préhension et la manipulation du logement par rapport au pavillon d'aiguille (28).

10. Système de placement de cathéter selon l'une quelconque des revendications précédentes, dans lequel le logement (110) inclut une fente de logement (116) s'étendant longitudinalement et communiquant avec l'un ou les deux parmi le canal d'aiguille (112) et le canal de fil-guide (114), la fente de logement étant configurée pour permettre au fil-guide (30) de passer à travers celle-ci lorsque le mécanisme de verrouillage (130) est en position non bloquée.

11. Système de placement de cathéter selon l'une quelconque des revendications précédentes, dans lequel le joint (120) inclut une fente de joint (126) s'étendant longitudinalement et communiquant avec l'une ou les deux parmi la lumière (124) de fil-guide de joint et une lumière (122) d'aiguille de joint, la fente de joint étant configurée pour permettre au fil-guide (30) de passer à travers celle-ci lorsque le mécanisme de verrouillage (130) est en position non bloquée.

12. Système de placement de cathéter selon la revendication 10 ou la revendication 11, dans lequel le verrou inclut une fente de verrou (136) s'étendant longitudinalement et communiquant avec une lumière (132) de verrou, la fente de verrou étant configurée pour s'aligner avec la fente de logement (116) dans la position non bloquée et permettre au fil-guide (30) de passer à travers celle-ci pour désolidariser le système de verrouillage de fil-guide.

13. Système de placement de cathéter selon l'une quelconque des revendications précédentes, dans lequel l'aiguille (20) inclut une ouverture de fil-guide (24) disposée dans une paroi latérale de l'aiguille et configurée pour recevoir une portion du fil-guide (30) à travers celle-ci, l'aiguille incluant également une fente d'aiguille (26) s'étendant longitudinalement entre l'ouverture de fil-guide et une pointe distale de l'aiguille et communiquant avec une lumière (22) d'aiguille, la fente d'aiguille étant configurée pour recevoir une portion du fil-guide (30) à travers celle-ci lorsque l'aiguille est retirée de manière proximale, et/ou
incluant en outre une gaine (70) entourant une portion de l'aiguille (20) et incluant une ligne de déchirure (72) s'étendant longitudinalement entre une extrémité proximale et une extrémité distale de la gaine.

14. Système de placement de cathéter selon l'une quelconque des revendications précédentes, dans lequel le verrou inclut une pièce de compression (142) couplée en rotation à une extrémité distale d'une pièce de rotation (144), la pièce de rotation étant configurée pour tourner autour d'un axe du canal de fil-guide (114) et pousser la pièce de compression axialement, et/ou
dans lequel la pièce de compression (142) inclut un rail (146) s'étendant à partir d'une surface externe de celle-ci et configuré pour venir en prise avec un évidement (148) disposé dans une paroi interne du canal de fil-guide (114) pour atténuer le mouvement de rotation de la pièce de compression tout en permettant un mouvement axial de celle-ci.

15. Système de placement de cathéter selon la revendication 1, dans lequel le mécanisme de verrouillage (130) inclut un actionneur (232) qui est en prise de manière coulissante avec le logement (110) le long d'un axe s'étendant perpendiculairement à un axe du canal de fil-guide (114), l'actionneur pouvant passer entre la position bloquée et la position non bloquée, facultativement
dans lequel le mécanisme de verrouillage (130) inclut un mécanisme à bouton-poussoir présentant une première force appliquée dans une première direction pour faire passer l'actionneur (232) de la position non bloquée à la position bloquée et une deuxième force également appliquée dans la première direction pour faire passer l'actionneur (232) de la position bloquée à la position non bloquée.
